# EUROPEAN PATENT SPECIFICATION

(11) **EP 2 979 664 B1**
(45) Date of publication and mention of the grant of the patent: **09.01.2019**
(21) Application number: 14179583.1
(22) Date of filing: 01.08.2014
(51) Int. Cl.: A61F 2/24

(54) **Aortic valve prosthesis, particularly suitable for transcatheter implantation**
Aortenklappenprothese, insbesondere zur Transkatheterimplantation
Prothèse valvulaire cardiaque, particulièrement appropriée à une implantation par transcathéter

(43) Date of publication of application: 03.02.2016
(73) Proprietor: Alvimedica Tibbi Ürünler Sanayi Ve Dis Ticaret A.S, 34540 Catalca, Istanbul (TR)
(72) Inventor: Minoletti, Francesco, 10149 Torino (IT); Antoniotti, Matteo, 10036 Settimo Torinese (Torino) (IT); Vallana, Franco, 10123 Torino (IT); Curcio, Maria, 13040 Saluggia (Vercelli) (IT)
(74) Representative: Gerbino, Angelo

(56) References cited:
- WO-A1-2013/059747
- WO-A1-2013/120181
- WO-A2-2011/002996
- US-A1- 2012 078 353

## Description

### Technical field

The present invention refers, in general, to heart valve prostheses; in particular, the invention relates to an aortic valve prosthesis.

### Background of the Invention

In the field of heart valve prostheses, with particular reference to aortic valves, solutions are being studied to implant such devices with minimally invasive techniques, to the benefit of the patient subject to the implantation.

In particular, new generation prosthetic aortic valves are designed for percutaneous implantation or minimally invasive surgical procedures. An example of transcatheter implantation is disclosed in document US 2002/0042651, concerning stented and unstented bioprosthetic valves, stented mechanical valves, and expandable or self-expanding valves, whether biological or artificial.

A first type of percutaneous valve prosthesis was provided with a frame comprising an anchoring element, adapted to support and fix the valve prosthesis firmly in the implantation site. A biocompatible artificial valve, able to reproduce the fluid dynamics of a native heart valve, is secured to such an anchoring element, which can be designed in the form of an annular structure consisting of one or more rings (e.g., as disclosed in US 4 056 854, describing an artificial valve including an expansible check valve remotely placeable in a blood vessel without major surgery to replace a malfunctioning natural valve; in US 5 370 685, which relates to a valve replacement system together with methods of preparation and use; in US 5 824 064, concerning a transfemoral aortic valve replacement with the simultaneous insertion of an aortic arch graft; and WO 02 41 789, which provides a valve configured for insertion on the proximal and distal sides of a heart valve annulus to replace the heart valve of a patient), in the form of an expandable stent with a reticular tubular wall (as disclosed in US 5 411 552, which describes a valve prosthesis for implantation by use of catheter, comprising a stent made from an expandable cylinder-shaped thread structure with several spaced apices; in EP 0 850 607, which is aimed to provide a valve prosthesis especially used in case of aortic stenosis, such structure being capable of resisting the powerful recoil force and to stand the forceful balloon inflation performed to deploy the valve and to embed it in the aortic *annulus;* in US 5 855 597, wherein a star-shaped stent and replacement valve or replacement graft for use in repairing a damaged cardiac valve includes two to eight star-shaped members interconnected into a "chain"; in US 5 855 601, describing an artificial heart valve which comprises a relatively rigid stent member having a first cylindrical shape and a flexible valve disposed in the stent member, the stent member being self-expandable to a second cylindrical shape and collapsible to its first cylindrical shape; in US 5 957 949, wherein the artificial valve includes a tubular graft having radially compressible annular spring portions for biasing proximal and distal ends of the graft into conforming fixed engagement with the interior surface of a generally tubular passage; in WO 00 41652, describing an apparatus and methods to improve the Boyden chamber used in cellular biological measurements, allowing quantitative optical microscopy of biological cells *in situ* without using fluorescent probes or optical staining; in WO 01 76510, which describes an expandable pre-assembled heart valve including a plastically-expandable annular base having a plurality of upstanding commissure posts, and a tubular flexible member including a prosthetic section and a fabric section; in US 6 482 228, wherein the prosthesis comprises a stent system expandable in the ascending aorta to anchor the valve in the aortic channel above the native aortic valve; and WO 03 003943, relating to improvements in prosthetic cardiac and venous valves and implantable medical devices having moveable septa, the inventive prosthetic cardiac and venous valves having metallic or pseudo-metallic valves coupled to metallic or pseudo-metallic stents that permit percutaneous delivery of the devices), or again in the form of an expandable stent consisting of a sheet or film wound in an optionally open-worked spiral (e.g., as disclosed in US 5 925 063, providing an apparatus which comprises a coiled sheet having a plurality of flaps mounted on its interior surface that project radially inward into a lumen formed by the interior surface of the apparatus when it is deployed, wherein the flaps lie parallel to the interior surface of the sheet during transluminal delivery, but project radially inward once the apparatus is deployed; and in WO 02076348, disclosing a two-piece stent which may include a primary stent to provide a tubular base at the annulus, and a secondary stent having the membranes that couples within the primary stent).

A second type of percutaneous cardiac-valve prostheses was instead provided with a set of prosthetic-valve structures, separated from an anchorage element and cooperating with it. The two entities are implantable in succession one after another, with the anchorage element set in position first. An example of said valve prosthesis with so-called "double structure" is represented by a stent in the form of a cage, with a function of support and anchorage of the valve, to which is associated a valve element in the form of an arrest ball (as described in US 5 332 402, wherein a cardiac valve is implanted within the heart while maintained in a collapsed form by cold temperature so as to percutaneously insert it along a releasable guide wire in a cooled sheath, until placed in position and expanded by withdrawing the cold temperature; and in US 5 397 351, disclosing a prosthetic valve which comprises a poppet, a seat and a restraining element, wherein in the operational form the poppet is movably restrained between the valve seat and the restraining element, in such a manner that the poppet seals against the valve seat to prevent fluid flow through the valve and unseats from the valve seat to permit fluid flow through the valve).

Other support and/or anchorage elements for valve prostheses, suitable for valve prostheses of a different nature, may have structures of the stent type (as described in WO 01 62189, relating to a device for fastening and anchoring cardiac valve prostheses which is essentially formed out of wire-shaped interconnected elements) or the structure of an annular type (as disclosed in EP 1 088 529, wherein the device, such as a ring for annuloplasty or a cardiac valve prosthesis comprises support formations of shape-memory material which can be wound on themselves or folded for the purpose of locating the device in the implant position, and a second, generally unfolded shape, to which the shape-memory material is brought after location of the support formations in the implant position).

Other embodiments of percutaneous cardiac-valve prostheses and corresponding methods of implantation are disclosed, for example, in US 4 777 951, describing a dilatation balloon to be introduced into a stenosed aortic valve via the aorta while the blood circulation of the patient is maintained by the heart via the aortic valve, the balloon being inflated to grossly deflect the leaflets of the valve in a manner avoiding blocking the outer portions of the commissures of the valve, so as to enable substantial flow of blood through the outer portions of the commissures during systole despite the presence in the valve of the large inflated balloon, and in US 5 954 766, wherein a device is controlled through pressure above a preselected threshold, the bulk resilience about a passageway in a valve body providing the mechanism for controlled flow.

In any case, the valve must be characterized by the ability to firmly anchor in the anatomical site, after being carried and delivered *in situ* by appropriate devices and catheters.

The native aortic valve can be subject to malfunctioning due to stenosis, calcification, hypertrophy, prolapse of the leaflets, etc. In such cases an aortic prosthesis can compensate for the functionality of the pathologic one. When implanted through percutaneous access, the prosthetic valve can be applied on the diseased structure, without the need for removal of the native valve.

If a surgical procedures is performed, the prosthetic valve is implanted in place of the native valve itself after its removal.

In order to enable a sufficiently firm anchoring of the prosthesis to the valve site, the prosthesis is provided with an armature in the form of a complex form with a longitudinal axis x, which has radially projecting formations, which formations engage lobed radial protrusions in the aorta, known as the sinuses of Valsalva.

Such anatomical structures are part of the artery, and not of the heart, so that the tissue on which the prosthesis is anchored is not subject to the same expansion and contraction rates as in the cardiac organ. This dimensional stability allows a proper anchorage of the prosthesis *in situ.*

An example of an aortic valve prosthesis, of the type described above, is disclosed in document EP 2319458 B1, wherein the artificial valve is supported by support pillars, secured to support rings. These rings are mutually connected by radially projecting formations, engaging the sinuses of Valsalva, thus allowing the anchoring of the prosthesis in place.

It is to be understood that the reference to any one of the preceding prior art documents must not be interpreted as limiting the scope of the present invention.

However, such a solution is fully effective only if the native valve is previously removed, so that such radially projecting formations are free to expand, until they engages the entire arched surface of the sinus of Valsalva.

In the case in which the native valve remains inside the site, the sinuses of Valsalva would not have a continuous surface, but instead would have a discontinuity in its profile, due to the calcific formations grown on the defective native valve.

In this way, the radially projecting formation is required to lay on a surface having a discontinuous profile, which implies, in the case of a formation consisting of a single filament, that the formation itself is not able to properly expand, to adapt completely to the sinus of Valsalva surface. Indeed, in the event that the axially lower part of the aortic valve site and the sinuses of Valsalva have a restricted section, due to calcifications, a lower portion of the radially projecting formation will lay on such restricted section, hindering the expansion of the its upper part, which shall not engage properly the rest of the sinus of Valsalva. This is because the lower part will put in traction the upper part of the radially projecting formation, holding it and preventing it from correctly and completely radially expand, thus weakening the anchoring force of the prosthesis on the valve site.

In order to overcome this drawback, radially projecting formations, mutually independent, would be required.

An example of an apparatus comprising such independent projecting formations is known from WO 2011/002996 A2. This document discloses a collapsible armature within which a prosthetic valve is placed. Such a valve is secured to axial support members, which expand from an elongated cylindrical configuration to a trapezoidal configuration when the armature is in a radially expanded configuration. The deformation causes the commissures (i.e., the structures connecting the valve to the support members) to undergo a certain stress, which could weaken the link between the valve and the armature. Moreover, the radial expansion causes a shortening of the armature in the axial direction, which could have an influence on the performances of the prosthesis.

WO 2013/059747 A1 discloses an apparatus comprising an armature with rigid pillars, which maintain the cylindrical configuration without undergoing any circumferential/axial deformation. Such pillars are shaped as rectangular foils or rectilinear rods. However, the formations projecting from the armature, in order to secure the prosthesis to the biological structure, are not suitable for a prosthesis to be used as an aortic valve.

### Summary of the invention

One purpose of the present invention is to overcome the above-mentioned problems, providing an aortic valve prosthesis that can be implanted through percutaneous access, ensuring a firm anchoring even when the native valve is not removed.

To achieve this result, the aortic valve prosthesis comprises an armature, including annular support elements which engage support pillars for the commissures of a prosthetic valve. Such support pillars are rectilinear Radially projecting formations, mutually independent, protrude from the annular support elements, being adapted to engage the sinuses of Valsalva, even in the presence of the native valve.

In particular, the lower radially projecting formations, connected to the lower annular support element, will be able to adapt to the inferior part of the Valsalva's sinuses pushing and anchoring the prosthesis to the native valve, even in presence of calcified formations. In fact, said lower radially projecting formations will benefit from an anchoring effect due to the fact that such formations will grasp to the calcified formations; in this way, the armature will be firmly secured in its lower part.

By contrast, the upper radially projecting formations, connected to the annular upper support, will be conveniently free to radially expand to an extent greater than that of the lower radially projecting formations, occupying in an optimal way the profile of the sinuses of Valsalva. This is possible thanks to the disconnection between the upper and lower radially projecting formations, in such a way that the hindered radial expandability of the latters does not affect the capacity of the formers to come effectively into contact with the inner surfaces of the sinuses of Valsalva.

Moreover, the upper and lower radially projecting formations may be axially spaced apart from one another, when in the expanded configuration, by a distance that allows them not to interfere with each other during the crimping steps, in which the armature is made to collapse to be placed *in situ,* where it will undergo radial expansion.

The above and other objects and advantages are achieved, according to one aspect of the invention, by an armature for an aortic valve prosthesis having the features defined in claim 1. Preferred embodiments of the invention are defined in the dependent claims.

### Brief description of the drawings

The structural and functional features of some preferred embodiments of an armature for an aortic valve prosthesis according to the invention are to be described in the following. Reference is made to the attached drawings, in which:
- Figure 1 is a schematic perspective view of an armature for an aortic valve prosthesis, according to one embodiment of the invention; and
- Figure 2 is a schematic axial section view of the armature of Figure 1, represented in an expanded condition.

### Detailed description

Before explaining in detail a plurality of embodiments of the present invention, it should be clear that the invention is not limited in its application to the details of construction and to the configuration of the components described in the following or illustrated in the drawings. The invention is able to assume other embodiments and of being implemented or realized practically in different ways.

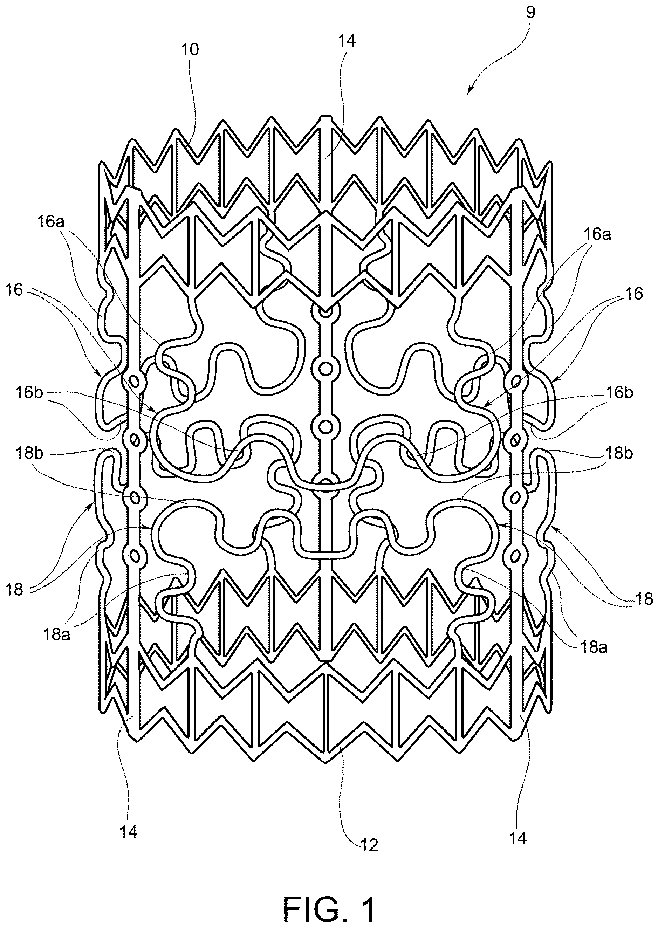

Referring initially to Figure 1, an armature 9 for an aortic valve prosthesis includes a pair of upper 10 and lower 12 annular support elements, axially spaced.

The annular support elements 10, 12 are collapsible and expandable from a contracted condition to a radially expanded condition, to allow the armature to be placed *in situ.*

Support pillars 14 are secured to the annular support elements 10, 12; the commissures of an artificial valve (not shown) are sewn to said support pillars 14.

A plurality of respective radially projecting formations 16, 18 protrude integrally with respect to each annular element 10, 12. Such formations, in an expanded condition of the armature 9, project radially from said armature to engage the sinuses of Valsalva. Conveniently, there will be an upper radially projecting formation 16 and a lower radially projecting formation 18 for each of the three sinuses of Valsalva.

Each radially projecting formation 16, 18 has, conveniently, a substantially annular shape, that comprises a pair of respective first and second meridian elements or filaments 16a, 18a, which may be circumferentially connected (as in the example shown herein below) via respective first and second circumferentially extending elements or filaments 16b, 18b. Alternatively, the first and second meridian elements or filaments 16a, 18a may have free ends (according to an embodiment not shown).

Said filaments 16a, 16b, 18a, 18b can have curved or zigzagging shapes (as illustrated herein).

The meridian filaments 16a, 18a may have a substantially axial orientation (i.e., parallel to a longitudinal axis x of the armature 9, when the radially projecting formations 16, 18 are in a radially contracted position), or may have a slight curvature, for example, a convexity or a camber (according to an embodiment not shown).

According to an alternative embodiment (not shown), each radially projecting formation 16, 18 may have more than two meridian filaments 16a, 18a, and/or more than one circumferentially extending element 16b, 18b, in such a way to form, for example, a grid instead of a ring.

In an expanded position, the upper radially projecting formations 16, integral with the upper annular support element 10, engage an upper portion of the inner surface of the sinuses of Valsalva; similarly, the lower radially projecting formations 18 engage a lower portion of the arched inner surface of the sinuses of Valsalva.

Since the upper 16 and lower 18 radially projecting formations are mutually independent, they will be able to expand in a radial direction up to engage in a proper manner the inner surfaces of the sinuses of Valsalva.

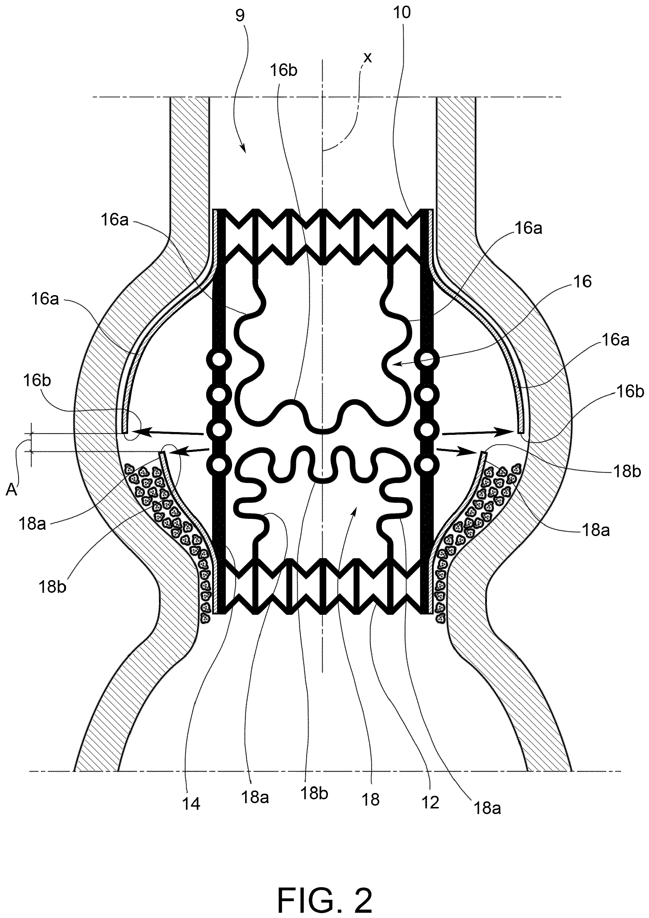

Indeed, as can be seen in particular in Figure 2, the lower 18 radially projecting formations can expand radially, engaging the calcified formations of the native valve in the valve site, while the upper radially projecting formations 16 are free to radially expand for an even greater extent, engaging the upper arched surfaces of the sinuses of Valsalva. This, as the lower radial expansion of the lower radially projecting formations 18 does not affect the possibility for the corresponding upper radially projecting formations 16 to expand further, since the two formations are mutually decoupled.

In this way, the armature 9 receives a proper anchoring to the biological formations of the aorta, without being affected by problems related to the placement *in situ* of the aortic valve prosthesis, if it were not possible to remove the nonfunctional native valve (for example, proceeding to a percutaneous implantation).

An axial distance A can be seen in figure 2, which axial distance separates the first circumferentially extending elements 16b (of the upper radially projecting formations 16) from the second circumferentially extending elements 18b (of the lower radially projecting formations 18). As already mentioned, said axial distance A allows, during the crimping step, to make the radially projecting formations collapse, without causing any interference between them or make them entangled with each other. This feature is therefore preferable, since adopting this approach avoids unwanted contacts or overlaps between the radially projecting formations, when the armature is made to collapse radially to be transported and placed *in situ.*

Various aspects and embodiments of the armature for a mitral valve prosthesis according to the present invention have been described. It is understood that each embodiment can be combined with any other embodiment. The invention, moreover, is not limited to the embodiments described, but can be varied within the scope of the invention as defined in the attached claims.

## Claims

1. An armature (9) for aortic valve prosthesis, said armature being radially expandable and collapsible, and comprising:
- an upper annular support element (10) and a lower annular support element (12);
- a plurality of support pillars (14), circumferentially spaced and axially extending, so as to connect said respective upper (10) and lower (12) annular support elements; and
- a plurality of radially projecting formations (16, 18), connected to the upper (10) and lower (12) annular support elements, said radially projecting formations (16, 18) being circumferentially spaced;
**characterized in that**:
said radially projecting formations (16, 18) are suitable to engage the sinuses of Valsalva, and comprise a plurality of upper radially projecting formations (16), connected to the upper annular support element (10), and a plurality of lower radially projecting formations (18), connected to the lower annular support element (12), so that the upper (16) and lower (18) radially projecting formations are mutually separated, and are independently expandable and contractible; and
the support pillars (14) are rectilinear.

2. An armature according to claim 1, wherein each upper radially projecting formation (16) comprises at least a pair of first meridian elements (16a) circumferentially spaced.

3. An armature according to claim 2, wherein first circumferentially extending elements (16b) circumferentially connect said first meridian elements (16a).

4. An armature according to any one of the preceding claims, wherein each lower radially projecting formation (18) comprises at least a pair of second meridian elements (18a) circumferentially spaced.

5. An armature according to claim 4, wherein second circumferentially extending elements (18b) circumferentially connect said second meridian elements (18a).

6. An armature according to any one of the preceding claims, wherein said first (16b) and second (18b) circumferentially extending elements are spaced apart from one another by an axial distance (A), when the respective radially projecting formations (16, 18) are in the radially expanded position.

7. An armature according to any one of the preceding claims, wherein said radially projecting formations (16, 18) have a filament-shaped or zigzagging curvilinear contour.

8. An aortic valve prosthesis, comprising an armature according to any one of the preceding claims.

## Patentansprüche

1. Verankerung (9) für eine Aortenklappenprothese, wobei die Verankerung radial expandierbar und zusammendrückbar ist und enthält:
- ein oberes ringförmiges Stützelement (10) und ein unteres ringförmiges Stützelement (12);
- mehrere Stützpfeiler (14), die in Umfangsrichtung beabstandet sind und sich axial erstrecken, so dass sie das jeweilige obere (10) und untere (12) ringförmige Stützelement verbinden; und
- mehrere radial vorspringende Gebilde (16, 18), die mit dem oberen (10) und unteren (12) ringförmigen Stützelement verbunden sind, wobei die radial vorspringenden Gebilde (16, 18) in Umfangsrichtung beabstandet sind;
**dadurch gekennzeichnet, dass**:
die radial vorspringenden Gebilde (16, 18) geeignet sind, die Sinus Valsalvae in Eingriff zu nehmen und eine Mehrzahl von oberen radial vorspringenden Gebilden (16) enthalten, die mit dem oberen ringförmigen Stützelement (10) verbunden sind, und eine Mehrzahl von unteren radial vorspringenden Gebilden (18) enthalten, die mit dem unteren ringförmigen Stützelement (12) verbunden sind, so dass die oberen (16) und unteren (18) radial vorspringenden Gebilde wechselweise getrennt sind und unabhängig voneinander entfaltbar und zusammendrückbar sind; und
die Stützpfeiler (14) geradlinig sind.

2. Verankerung nach Anspruch 1, wobei jedes der oberen radial vorspringenden Gebilde (16) zumindest ein Paar von ersten Meridianelementen (16a) enthält, die in Umfangsrichtung beabstandet sind.

3. Verankerung nach Anspruch 2, wobei die ersten sich in Umfangsrichtung erstreckenden Elemente (16b) in Umfangsrichtung die ersten Meridianelemente (16a) verbinden.

4. Verankerung nach einem der vorhergehenden Ansprüche, wobei jedes untere radial vorspringende Gebilde (18) zumindest ein Paar von zweiten Meridianelementen (18a) enthält, die in Umfangsrichtung beabstandet sind.

5. Verankerung nach Anspruch 4, wobei zweite sich in Umfangsrichtung erstreckende Elemente (18b) in Umfangsrichtung die zweiten Meridianelemente (18a) verbinden.

6. Verankerung nach einem der vorhergehenden Ansprüche, wobei die ersten (16b) und zweiten (18b) sich in Umfangsrichtung erstreckenden Elemente voneinander um einen axialen Abstand (A) beabstandet sind, wenn die jeweiligen radial vorspringenden Gebilde (16, 18) in der radial expandierten Position sind.

7. Verankerung nach einem der vorhergehenden Ansprüche, wobei die radial vorspringenden Gebilde (16, 18) eine filamentförmige oder zickzackartige gekrümmte Kontur aufweisen.

8. Aortenklappenprothese, die eine Verankerung nach einem der vorhergehenden Ansprüche enthält.

## Revendications

1. Armature (9) pour une prothèse valvulaire aortique, ladite armature étant radialement déployable et rétractable, et comprenant :
- un élément de support annulaire supérieur (10) et un élément de support annulaire inférieur (12) ;
- une pluralité de piliers de support (14), circonférentiellement espacés et s'étendant axialement, de manière à raccorder lesdits éléments de support annulaires supérieur (10) et inférieur (12) respectifs ; et
- une pluralité de formations se projetant radialement (16, 18), raccordées aux éléments de support annulaires supérieur (10) et inférieur (12), lesdites formations se projetant radialement (16, 18) étant circonférentiellement espacées ;
**caractérisé en ce que** :
lesdites formations se projetant radialement (16, 18) sont adaptées à mettre en prise les sinus de Valsalva, et comprennent une pluralité de formations se projetant radialement supérieures (16), raccordées à l'élément de support annulaire supérieur (10), et une pluralité de formations se projetant radialement inférieures (18), raccordées à l'élément de support annulaire inférieur (12), de telle sorte que les formations se projetant radialement supérieures (16) et inférieures (18) sont mutuellement séparées, et sont indépendamment déployables et contractables ; et
les piliers de support (14) sont rectilignes.

2. Armature selon la revendication 1, dans laquelle chaque formation se projetant radialement supérieure (16) comprend au moins une paire de premiers éléments méridiens (16a) circonférentiellement espacés.

3. Armature selon la revendication 2, dans laquelle les premiers éléments s'étendant circonférentiellement (16b) raccordent circonférentiellement lesdits premiers éléments méridiens (16a).

4. Armature selon l'une quelconque des revendications précédentes, dans laquelle chaque formation se projetant radialement inférieure (18) comprend au moins une paire de seconds éléments méridiens (18a) circonférentiellement espacés.

5. Armature selon la revendication 4, dans laquelle les seconds éléments s'étendant circonférentiellement (18b) raccordent circonférentiellement lesdits seconds éléments méridiens (18a).

6. Armature selon l'une quelconque des revendications précédentes, dans laquelle lesdits premiers (16b) et seconds (18b) éléments s'étendant circonférentiellement sont espacés les uns des autres d'une distance axiale (A), lorsque les formations se projetant radialement (16, 18) respectives sont dans la position radialement déployée.

7. Armature selon l'une quelconque des revendications précédentes, dans laquelle lesdites formations se projetant radialement (16, 18) ont un contour en forme de filament ou curviligne en zigzags.

8. Prothèse valvulaire aortique, comprenant une armature selon l'une quelconque des revendications précédentes.
